**Europäisches Patentamt**

**European Patent Office**

**Office européen des brevets**

(19)

(11) Veröffentlichungsnummer: **0 395 717 B1**

(12)

# EUROPÄISCHE PATENTSCHRIFT

(45) Veröffentlichungstag der Patentschrift: **29.09.93**

(21) Anmeldenummer: **89901258.7**

(22) Anmeldetag: **11.01.89**

(86) Internationale Anmeldenummer:
**PCT/DE89/00010**

(87) Internationale Veröffentlichungsnummer:
**WO 89/06518 (27.07.89 89/16)**

(51) Int. Cl.5: **A61B 17/36**, A61C 1/00,
B23K 26/00

(54) **VORRICHTUNG ZUR ABLATIVEN PHOTODEKOMPOSITION VON ORGANISCHEN UND ANORGANISCHEN SUBSTANZEN, INSBES. ZAHNHARTSUBSTANZEN.**

(30) Priorität: **12.01.88 DE 3800555**

(43) Veröffentlichungstag der Anmeldung:
**07.11.90 Patentblatt 90/45**

(45) Bekanntmachung des Hinweises auf die
Patenterteilung:
**29.09.93 Patentblatt 93/39**

(84) Benannte Vertragsstaaten:
**AT BE CH DE FR GB IT LI LU NL SE**

(56) Entgegenhaltungen:
EP-A- 0 111 060      EP-A- 0 152 686
EP-A- 0 194 856      EP-A- 0 196 519
US-A- 3 528 424      US-A- 3 658 406
US-A- 4 408 602      US-A- 4 501 993
US-A- 4 686 979

(73) Patentinhaber: **Dardenne, Michael Ulrich, Prof. Dr. Dr.**
**Graf Stauffenberg-Str. 36**
**D-53115 Bonn(DE)**

(72) Erfinder: **DARDENNE, Michael Ulrich, Prof.Dr.**
**Graf-Stauffenberg-Strasse 36**
**D-5300 Bonn 1(DE)**
Erfinder: **KERMANI, Omid, Dr.**
**Karthäuser Strasse 13**
**D-5300 Bonn 1(DE)**
Erfinder: **KOORT, Hans-Joachim, Dr.**
**An St. Josef 29 a**
**D-5300 Bonn 3(DE)**

(74) Vertreter: **Koch, Theodor, Dipl.-Phys.**
**Postfach 19 01 26**
**D-53037 Bonn (DE)**

EP 0 395 717 B1

**Beschreibung**

Die Erfindung bezieht sich auf eine Vorrichtung zur ablativen Photodekomposition von biologischen Substanzen, welche einen gepulsten Argon/Fluorid-Excimer-Laser aufweist, der mit einer Impulsdauer von 8 - 35 nsec UV-Strahlung einer Wellenlänge von 193 nm bei einer Impulsrate kleiner als 100 Hz emittiert und eine Ausgangsimpuls-Energiedichte von mindestens 50 mJ/cm$^2$ besitzt, sowie mit einer Applikationsvorrichtung zur Leitung der UV-Strahlung des Excimer-Lasers zum Applikationsbereich, wobei das Laserlicht mittels der Applikationsvorrichtung über einen Applikationsarm, in welchem ein Spiegel- und Linsensystem für das UV-Licht angeordnet ist, auf den Applikationsbereich unter Fokussierung auf eine Fläche von kleiner/gleich 2 mm$^2$ geleitet ist.

Eine derartige Vorrichtung zur ablativen Photodekomposition von biologischem Gewebe ist gemäß der EP-A1-0 111 060 bekannt. Es soll dabei generell zum Abtragen von biologischem Gewebe in Form von organischen Eiweiß-Schichten Laser-UV-Strahlung mit einer Wellenlänge geringer als 200 nm benutzt werden, wobei insofern auch die Verwendung eines Argon/Fluorid-Excimer-Lasers vorgeschlagen wird.

Dabei soll ohne Aufheizung der bestrahlten biologischen Schichten lediglich weiches organisches Material, wie kariös veränderter Zahnschmelz, bei geringen Impuls-Energiedichten abgetragen werden. Im Behandlungsbereich sollen diese nur im Bereich von 10 - 1000 mJ/cm$^2$/Impuls liegen. Die Applikation des Laserlichtes soll dabei gemäß dem US-Patent 3,821,510 über eine flexible Laserstrahlfaser erfolgen, welche unmittelbar in der Hand gehalten werden kann. Ferner ist die Verwendung eines Gehäuses vorgesehen, welches mit Stickstoffgas gespült werden soll, um die Absorption der Laser-UV-Strahlung von 193 nm durch Sauerstoff zu verhindern. Der kollimmierte Strahl des Laserlichtes soll dabei entweder über eine Linse auf den Behandlungsbereich abgebildet werden oder unmittelbar durch die Verwendung einer Schlitzmaske auf den zu bestrahlenden, auszuwählenden Bereich des organischen Materials eingestrahlt werden.

Aufgrund der insofern niedrigen applizierten Impuls-Energiedichten wird bei der Verwendung der Laser-UV-Strahlung zur Zahnbehandlung lediglich zunächst kariös veränderter Zahnschmelz in Form von reinem organischen Material abgetragen, wobei des weiteren die Abtragung des darunter liegenden organischen biologischen Materials in Form von Dentin möglich ist. Die Abtragung von gesundem Zahnschmelz, in welchem anorganische Substanzen, wie CaF, einen wesentlichen Bestandteil bilden, ist dabei nicht möglich und auch nicht gewollt.

Auf diese Weise kann zwar mit großer Genauigkeit eine Beseitigung der kariös veränderten Zahnsubstanzen vorgenommen werden, wobei gesunder Zahnschmelz und auch die die Wurzeln des Zahnes umgebenden Knochensubstanzen nicht abgebaut werden, andererseits kann aber bei diesen geringen Impuls-Energiedichten eine an sich ggf. gewünschte Präparation der Oberflächen von gesunden Zahnhartsubstanzen nicht vorgenommen werden. Insofern muß dies in üblicher mechanischer Weise erfolgen oder mittels solcher Laser, welche bisher bereits in der Zahnbehandlung Anwendung gefunden haben, und im sichtbaren und/oder infraroten Wellenlängenbereich durch bloße "Verbrennung" der abzutragenden Zahnsubstanzen arbeiten (Nd-YAG-Laser).

Eine Photoablation ist bei derartigen Lasern, wie auch bei einem $CO_2$-Laser, welcher vor allen Dingen wegen seiner geringen Eindringtiefe in das Gewebe benutzt wird, und welcher ein sehr exaktes Schneidinstrument darstellt, um ein mikrochirurgisches Arbeiten bzw. flächenhaftes Abtragen vorzunehmen, aufgrund des geringen zur Verfügung stehenden Intensitätbereiches der Strahlung dieser Laser nicht möglich.

Trotz dieser geringen Intensitäten tritt bei diesen Lasern beim Abtragen von Zahnhartsubstanzen und auch generell bei biologischen organischen und anorganischen Substanzen eine erhebliche Erwärmung auch der tieferen an sich nicht abzutragenden Gewebebereiche ein. Es ist dabei keine exakte Unterscheidung zwischen einzelnen Gewerbearten, insbesondere zwischen einzelnen unterschiedlichen Zahnhartsubstanzn möglich, wobei insbesondere zwangsweise mit Abtragung von kariösem Dentin oder Zahnschmelz auch angrenzende gesunde organische Zahnhartsubstanzen durch den Verbrennungsprozeß mit abgetragen werden. Aufgrund dieser Ungenauigkeiten läßt sich mit diesen bekannten Lasern kein genaues Schnittprofil in den Zahnhartsubstanzen erzeugen, wobei seitlich in den Schnittflächen sich Aushöhlungen ergeben. Insofern ist auch ein Konditionieren der zu behandelnden Oberflächen der Zähne nicht in der an sich notwendigen Weise möglich.

Durch die bei diesen mit Verbrennung des abzutragenden Zahnmaterials arbeitenden Lasern sich einstellenden hohen Temperaturen im Zahnmaterial ergeben sich, wie bei der Abtragung von Zahnhartsubstanzen durch elektrisch angetriebene mechanische Bohrer, unterhalb der Bearbeitungszone Beschädigungen des Zahnes in Form von Trümmerzonen oder durch die Bildung von Mikrorissen. Es ist dabei nicht möglich, die Zahnhartsubstanzen lediglich in Schichtdicken kleiner als 1 Micron oder lediglich in Form äußerst dünner Zahnsteinbeläge abzutragen. Auch bei Abtragungen von Zahnhartsubstanzen außerhalb des eigentlichen Wurzelbereiches des Zahnes ergeben sich dabei zwangsweise durch die große Temperaturerhitzung des Zahnes erhebliche Schmerzen für den Patienten während der Zahnbehandlung.

Der Erfindung liegt daher die Aufgabe zugrunde, eine Vorrichtung zum Abtragen von sowohl organischen als auch anorganischen Gewebesubstanzen zu schaffen, wobei die unterschiedlichsten Gewebesubstanzen und -arten, insbesondere Zahnsubstanzen, wie bloßer Zahnstein, Dentin und kariös veränderter Zahnschmelz, aber auch Zahnhartsubstanzen, wie gesunder Zahnschmelz,
in kontrollierter Weise, genau und in geringen Schichtdicken für sich jeweils einzeln abbaubar sind, wobei keine größeren thermischen oder mechanischen Belastungen der gesunden oder nicht zu behandelnden Gewebearten und Zahnhartsubstanzen auftreten. Bei letzteren sollen sich insbesondere unter den Bearbeitungszonen keine Trümmerzonen, Mikrorisse und dgl. ergeben.

Zur Lösung dieser Aufgabe ist bei der eingangs genannten Vorrichtung zur ablativen Photodekomposition von biologischen Substanzen vorgesehen,
daß zur Ermöglichung einer ablativen Photodekomposition von anorganischen Zahnhartsubstanzen im Fokus eine Impuls-Energiedichte von mindestens 2500 mJ/mm$^2$ erzeugt ist.

In einfachster Weise wird somit durch die eingestrahlte äußerst kurzwellige Strahlung eines Argon/Fluorid-Excimer-Lasers von 193 nm, welche nahezu verlustfrei über die Applikationsvorrichtung mit einer äußerst hohen Impuls-Energiedichte von mindestens 2500 mJ/mm$^2$ appliziert wird, erreicht, daß unmittelbar mit der selben Vorrichtung in gewünschter Weise organische und/oder anorganische Substanzen durch ablative Photodekomposition von Materialfragmenten aus deren Oberfläche abgetragen werden. Dies kann dabei in kontrollierter Weise, genau und in geringen Schichtdicken erfolgen.

Gemäß der Erfindung wird dabei insbesondere die Abtragung von anorganischen Zahnhartsubstanzen durch ablative Photodekomposition mittels UV-Strahlung einer Wellenlänge von 193 nm ermöglicht.

Dieses Verfahren läßt sich dabei in kontrollierter Weise jeweils für die einzelnen Schichtdicken der unterschiedlichen Zahnsubstanzen durchführen, wobei keine thermischen oder mechanischen Belastungen auftreten. Da die Abtragungsraten für kariöses Dentin um einen Faktor von 10 - 100 gegenüber gesundem Dentin oder Zahnschmelz höher liegen, ist es möglich, die kranken Zahnsubstanzen abzutragen, ohne dabei die gesunden anzugreifen. Eine Unterscheidung dieser Zahnsubstanzen ist dabei aufgrund der unterschiedlichen Färbung oder sich dort ergebender unterschiedlicher Fluoreszenz von eingestrahltem Licht möglich.

Durch die ultraviolette Laserstrahlung von 193 nm lassen sich insbesondere auch chemisch aktive Oberflächen an den Zahnsubstanzen insofern herstellen, als durch die Photoablation die abzutragenden Schichten automatisch vollkommen entfernt werden, so daß keine Nachbehandlung notwendig ist. Dagegen mußte bisher die entsprechende Stelle des Zahnes mit Phosphorsäure aufgerauht werden, um chemisch aktive Oberflächen zu erzeugen.

Diese Phosphorsäure dringt zum einen in Mikrorisse des Zahnes ein und vergrößert diese Mikrorisse damit nachteilig. Zum anderen bilden die Reaktionsprodukte ein Monolayer mit Wasser, was zur Spülung der Zähne verwendet wird. Diese Lage verhindert dann ein sicheres Ankleben der Adhäsivsysteme.

Aufgrund der hohen Impuls-Energiedichte im Fokus des Applikationsbereiches können dabei bei Behandlung von Zahnsubstanzen sowohl organisches Zahnmaterial, wie z. B. Dentin, und anorganisches Zahnmaterial, bzw. derartige in einer organischen Matrix eingelagerte Substanzen abgebaut werden. Dies, da ab einem bestimmten Minimalwert der Impuls-Energiedichte im Fokus gleichzeitig sowohl organische als auch anorganische Zahnsubstanzen abgebaut werden, allerdings mit unterschiedlichen Abtragungsraten. Das Abtragen von gesundem Zahnschmelz, welcher in seiner organischen Matrix anorganische Bestandteile in Form von CaF aufweist, ist dabei in der Zahnmedizin insofern von Bedeutung, um z. B. ein "Konditionieren" der Oberfläche des gesunden Zahnschmelzes zu erreichen, von welcher der kariöse Zahnschmelz oder kariöses Dentin abgetragen ist, so daß chemisch aktive, trümmerfreie Oberflächen an den präparierten Stellen des Zahnes entstehen und somit eine sichere Einbringung von Füllungen und anderen Adhäsiv-Systemen möglich ist. Die Erfindung benutzt dabei die Feststellung, daß die Abtragungsraten für kariöses Dentin und Zahnschmelz um einen Faktor von 10 - 100 gegenüber den Abtragungsraten von gesundem Dentin oder Zahnschmelz höher liegen, so daß trotz möglicherweise gleichzeitiger Bestrahlung unterschiedlicher Zahnhartsubstanzen und reiner biologischer organischer Substanzen (wie kariöses Dentin oder kariöser Zahnschmelz) Abtragungen in geeigneten unterschiedlichen Dicken erreichbar sind, und aufgrund der unterschiedlichen Färbung dieser Substanzen eine spezifische Abtragung in geringer Schichtdicke unter Beseitigung der kariös veränderten Bereiche möglich ist.

Die Applikation des Laserlichtes ist dabei ohne die Verwendung von Glas- oder Quarzfasern möglich, welche als solche die Strahlung von 193 nm in hohem Maße absorbieren und im übrigen auch bei Einstrahlung hoher Ausgangsimpuls-Energiedichten durch die 193 nm Laserstrahlung zerstört werden. Es wird dabei eine Applikationsvorrichtung mit einem Applikationsarm in Form eines herkömmlichen mechanischen Bohrers vorzugsweise verwendet, welcher gegenüber der äußeren Atmosphäre abgedichtet und evakuiert ist. Der Applikationsarm wird dabei mit Stickstoff unter Kühlung gespült, wobei in diesem selbst ein Spiegel- oder Linsensystem angeordnet ist, dessen Material für die Reflektion und Transmission von UV-Licht der Wellenlänge von 193 nm optimiert ist und dabei eine Fokussierung der Laser-UV-Strahlung mit hoher Impuls-Energiedichte auf einen kleinen Fokus von weniger als 1 - 2 mm$^2$ ermöglicht. Es ist dabei ein Betrieb der gepulsten Vorrichtung möglich, ohne daß das Spiegel- und Linsensystem durch die hohe Leistung von etwa 10 Megawatt/Impuls ausgestrahlter Laserenergie zerstört wird. Über kommerziell erwerbbare Argon/Fluorid-Excimer-Laser mit einer Ausgangsimpuls-Energiedichte von 200 mJ/cm$^2$ ist es dabei möglich, äußerst schonend und spezifisch die unterschiedlichen Zahnhartsubstanzen abzutragen, sobald ein bestimmter "Schwellenwert" der effektiven Impulsenergie-Dichte der ultravioletten Laser-Strahlung von 193 nm für die abzutragenden unterschiedlichen Materialien jeweils überschritten ist. Es ist dabei möglich, eine nahezu schmerzfreie Zahnbehandlung unter Präparation bzw. Abtragung gesunder und kariös veränderter Zahnkantsubstanzen vorzunehmen, sofern die Bearbeitungszonen außerhalb der den Zahnnerv führenden Zonen liegen.

Mit der erfindungsgemäßen Applikationsvorrichtung läßt sich somit die zur Zeit etwa lediglich maximal mit Ausgangsimpuls-Energiedichten von etwas mehr als 400 mJ/cm$^2$ zur Verfügung stehende 193 nm Laserstrahlung eines Argon/Fluorid-Excimer-Lasers mit ausreichender Effektivität in der Zahnbehandlung nutzen. Bei Repetitionsraten des Laserimpulses unter 100 Hz ist es dabei möglich, die Leistung der ausgestrahlten Laserenergie-Impulse in ausreichender Größenordnung von 10 Megawatt/Impuls zu erhalten, wobei eine Fokusfläche in der Größenordnung von 1 - 2 mm$^2$ erzeugbar ist. Bei einer zur Verfügung stehenden effektiven Ausgangsimpuls-Energiedichte von 100 mJ/cm$^2$ des verwendeten Lasers ist es dabei möglich, die effektive Impuls-Energiedichte bei der genannten Größe der Fokusfläche auf 5000 mJ/mm$^2$ zu erhöhen. Bei dieser Energie läßt sich dabei pro Laserimpuls mit einer Dauer von 15 - 20 nsec in dichter Hydroxylapatit-Keramik eine Schichtdicke von 0,14 Micron abtragen. Die Abtragungsrate liegt dabei bei kariösem Dentin um einen Faktor 10 - 100 höher. Der Handgriff der Applikationsvorrichtung erlaubt dabei eine Verstellbarkeit um zwei Drehachsen und um eine Schwenkachse, so daß eine gute Handbarkeit sich ergibt. Das Gewicht dieses Handgriffes und dessen Größenabmessung kann dabei in der Größenordnung üblicher elektronisch angetriebener, mechanischer Bohrvorrichtungen zur Zahnbehandlung gehalten werden.

Vorteilhafte Ausführungsformen der Vorrichtung zur ablativen Photodekomposition von Zahnhartsubstanzen, sowie der dabei verwendeten Applikationsvorrichtung für die 193 nm ultraviolette Laserstrahlung des zu verwendenden Argon/Fluorid-Excimer-Lasers ergeben sich aus der folgenden Beschreibung von Ausführungsformen der Erfindung mit Bezug auf deren Zeichnung.

Im folgenden wird die Erfindung anhand zweier bevorzugter Ausführungsbeispiele unter Bezug auf deren Zeichnungen näher erläutert.

In den Zeichnungen zeigen:

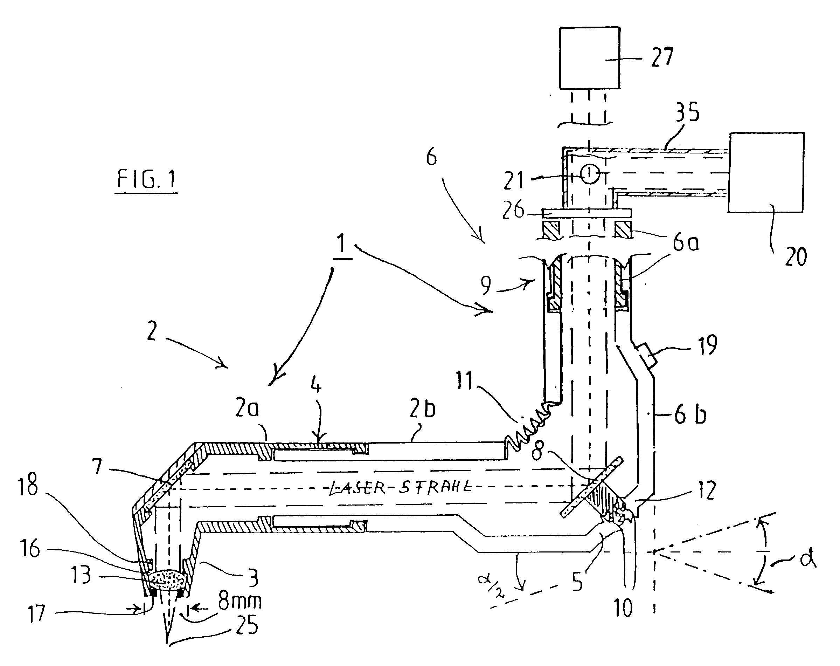

Figur 1: Die erste Ausführungsform einer Applikationsvorrichtung mit Handgriff zum nahezu verlustfreien Einspiegeln und zum Applizieren der Laserstrahlung des eine Wellenlänge von 193 nm emittierenden Argon/Fluorid-Excimer-Lasers unter schematischer Darstellung des Strahlenganges und der Anordnung dieses Excimer-Lasers und eines Ziel-Lasers;

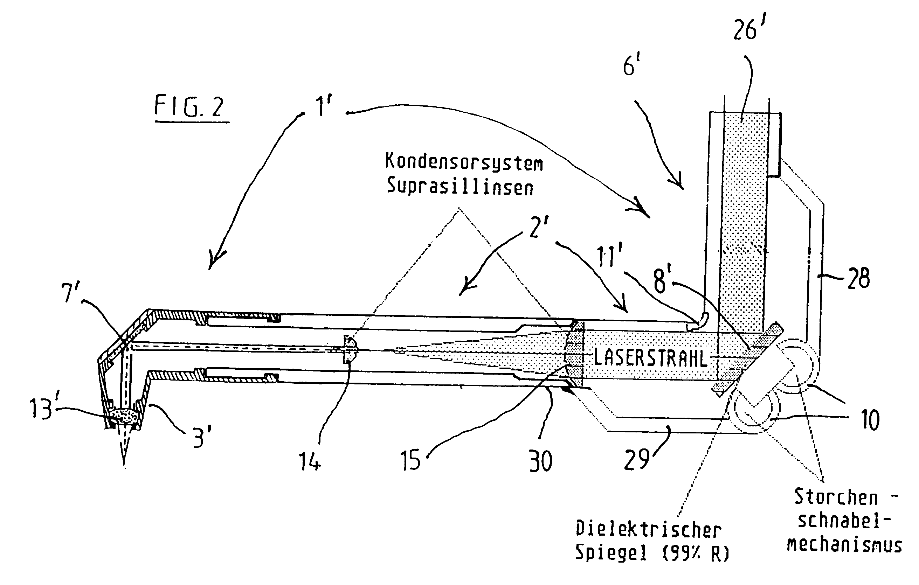

Figur 2: Eine zweite Ausführungsform der Applikationsvorrichtung zum Einspiegeln und zum Applizieren der Laserstrahlung des Argon /Fluorid-Excimer-Lasers (193 nm) zur ablativen Photodekomposition von Zahnhartsubstanzen, in einem Aufbau entsprechend der Applikationsvorrichtung gemäß Figur 1, wobei der erste rohrförmige, zu einem Optikkopf am linken Anfang abgewinkelte Griffabschnitt eine nach außen geschlossene Mantelfläche aufweist, in welchem ein zusätzliches Kondensorsystem aus Suprasillinsen angeordnet ist, und der zweite Griffabschnitt offen ist. Seitlich dieser Griffabschnitte ist ein Schwenkmechanismus in Form eines nach außen offenen Storchenschnabelmechanismusses angelegt, dessen beide Schenkel sich immer gleichzeitig derart öffnen, daß auf einen im Drehpunkt der Schwenkvorrichtung angeordneten dielektrischen Spiegel der Reflektionspunkt des Laserstrahles jeweils an der gleichen Stelle erscheint;

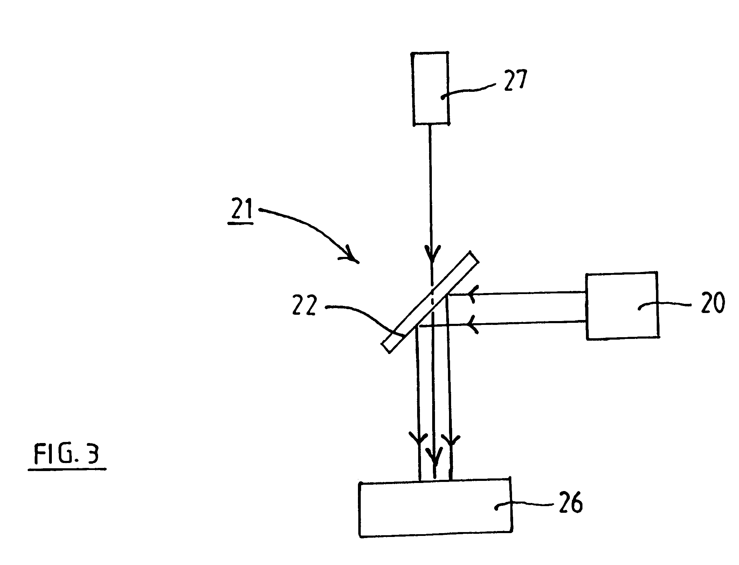

Figur 3: Die schematische Darstellung eines Einkoppelmechanismusses zum gleichzeitigen oder alternativen Einspiegeln der Laserstrahlung eines Argon/Fluorid-Excimer-Lasers , welcher UV-Strahlung (193 nm) emittiert, sowie eines sichtbares Licht emittierenden Lasers (HeNe-Lasers) unter Verwendung eines dielektrischen Spiegels, wobei die UV-Strahlung bzw. sichtbare Strahlung in den Eingang der Applikationsvorrichtung gemäß Figur 1 bzw. Figur 2 fällt:

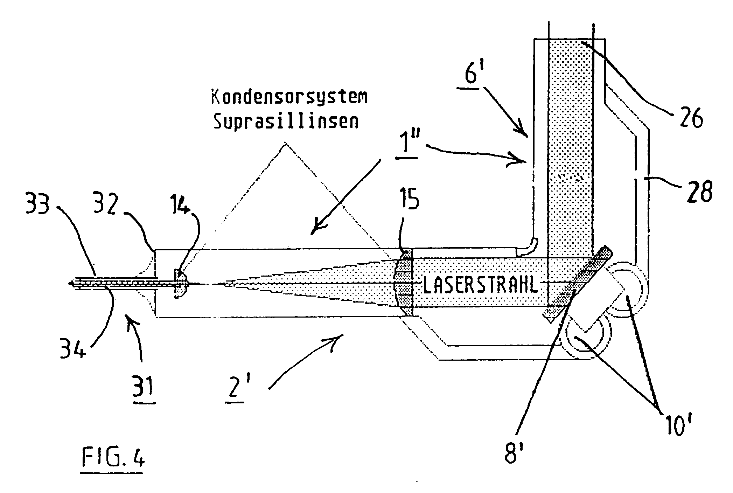

Figur 4: Eine Abänderung der Ausführungsform gemäß Figur 2.

Den prinzipiellen Aufbau eines Ausführungsbeispieles der Vorrichtung zur ablativen Photodekomposition von Zahnhartsubstanzen in gesunden oder auch kariös veränderten Bereichen der Zähne erkennt man in der schematischen Darstellung der Figur 1.

Danach weist diese Vorrichtung einen eine Wellenlänge von 193 nm emittierenden Argon/Fluorid-Excimer (20) auf, aus dessen für kurzwellige UV-Strahlung durchlässiges Quarzausgangsfenster über eine evakuierte Glasröhre die UV-Strahlung in den Eingang (26) einer fokussierhandstückartiken Applikationsvorrichtung (1) zum Einspiegeln und zum Applizieren der Laserstrahlung auf eine Fokusfläche (25) in dem aufzulösenden Bereich einer Zahnhartsubstanz appliziert wird.

In der Fokusfläche (25) besitzt die von dem Argon/Fluorid-Excimer-Laser (20) emittierte ultraviolette Laserstrahlung gegenüber der Ausgangsimpuls-Energiedichte des Lasers von 200 mJ/cm$^2$ eine bedeutend erhöhte Impuls-Energiedichte von mindestens 5000 mJ/mm$^2$, wobei der Fokuspunkt kleiner gleich 2 mm$^2$ ist. Diese ultraviolette Laserstrahlung wird dabei mit einer Impulsrate von 25 Hz eingestrahlt, wobei die auf diese Weise übertragene Energie des Laserstrahles so hoch ist, daß die ca. 6,4 eV Photonenergie aufgrund der hohen Energiedichte der Laserimpulse Mehrphotonenprozesse auslösen, so daß die Photonenenergien der Laserstrahlung sich auf Größenordnungen kumulieren, welche im Bereich der organischen Zahnhartsubstanzen, seien diese nun gesund oder kariös, liegen (10 - 15 eV).

Es ist insofern eine äußerst schonende ablative Photodekomposition der Zahnhartsubstanzen möglich, wobei bei einer effektiven Impuls-Energiedichte von 100 mJ/cm$^2$ es möglich ist, je Impuls bei einer Fokusfläche von Kleiner/gleich 2 mm$^2$ eine Abtragung von 0,14 Micron in dichter Hydroxylapatitkeramik bzw. in gesundem Dentin bei 5000 mJ/mm$^2$ vorzunehmen. Da die Abtragungsrate dagegen bei kariösem Dentin um einen Faktor von 10 - 100 höher liegt, und dabei das kariöse Dentin gegenüber dem gesunden Dentin bzw. Zahnschmelz durch seine Färbung oder eine unterschiedliche Fluoreszenz des Lichtes eines Zielstrahles unterscheidbar ist, läßt sich das kariöse Dentin durch den Laserstrahl abtragen, ohne dabei den gesunden Zahnschmelz oder das gesunde Dentin anzugreifen. Die Zahnhartsubstanzen werden dabei durch Auflösung der atomaren Bindungskräfte in den Hydroxylapatitkristalliten abgetragen, wobei dies ohne Verbrennung des Zahnmaterials geschieht. Gleichzeitig vorgenommene Temperaturmessungen haben dabei ergeben, daß bei einfacher Kühlung durch Luftstrom lediglich eine maximale Temperatur-Erhöhung des Zahnmaterials von 2° erfolgt.

Im Gegensatz zu dieser Abtragung ist bei bisher verwendeten Lasern in der Zahnheilkunde, welche im sichtbaren oder infraroten Wellenlängenbereich emittieren (z. B. Nd: YAG), lediglich ein Abbau der Zahnhartsubstanzen durch Erwärmung oder Erhitzen, also durch Verbrennung von Zahnhartsubstanzen an den zu behandelnden Stellen möglich.

Es ist somit möglich, aufgrund der ablativen Photodekomposition äußerst gerade senkrechte Schnittprofile und feinste Abtragungen im Bereich eines Bruchteils eines Mikrometers zu erzeugen, wobei keine Aushöhlungseffekte an den Abtragungsbereichen sich ergeben.

Da mit der ablativen Photodekomposition keine mechanischen Erschütterungen der Zahnhartsubstanzen in der Bearbeitungszone verbunden sind, ergeben sich im Gegensatz zu konventionellen Methoden keine Trümmerzonen, Mikrorißbildungen oder andere mechanischen Beschädigungen der Zahnhartsubstanzen. Es ist dabei möglich, durch die kurzwellige UV-Strahlung des Argon/Fluorid-Excimer-Lasers die Abtragung von Zahnhartsubstanzen ohne Vibrations-, Deformations- und Reibungseffekte durchzuführen, so daß sich eine äußerst schmerzarme Zahnbehandlung ergibt. Es läßt sich dabei insbesondere auch Zahnstein von gesundem oder kariösem Dentin bzw. Schmelz entfernen.

Ferner sind gute Voranssetzungen für die Präparation der Zahnhartsubstanzen zur Versorgung des Zahnes mit Adhäsivsystemen gegeben, da durch die UV-Strahlung von 193 nm an der Oberfläche der Zahnhartsubstanzen chemisch aktive, trümmerfreie Flächen entstehen. Außerdem können nunmehr auch an gesunden Zähnen, ohne große Schäden anzurichten, Verankerungen für Brücken und sonstige zahnerhaltende Maßnahmen angebracht werden.

Da zur Unterscheidung von kariösem Dentin und gesundem Dentin an sich die unterschiedliche Färbung dieser Zahnsubstanzen genügt, weist die Ausführungsform der Vorrichtung zur ablativen Photodekomposition von Zahnhartsubstanzen gemäß Figur 1 und Figur 3 bzw. Figur 2 und Figur 3 keine besondere Steuervorrichtung zum Ein- und Ausschalten des Argon/Fluorid-Excimer-Lasers (20) auf, wie dies an sich über ein Spektralanalyse-System erfolgen kann, welches die Änderung der Fluoreszenz während des Überganges von kariösem auf gesunden Zahnhartsubstanzen mißt und damit den Excimer-Laser (20) bzw. dessen Laserstrahlung ausschaltet.

Die Ein- und Ausschaltung des Argon/Fluorid-Excimer-Lasers (20) bzw. des als Ziel-Laser verwendeten Helium-Neon-Lasers (27) erfolgt dabei lediglich über einen am Handgriff (1) selbst angebrachten Ein/Ausschalter (19).

Über den Eingang (26) für die durch den Handgriff (1) zu applizierende Laserstrahlung kann dabei außer der Laserstrahlung des Argon/Fluorid-Excimer-Lasers (20) die Strahlung eines im sichtbaren Wellenlängenbereich (z. B. rotes Licht) emittierenden Ziel-Lasers (27) eingestrahlt werden. Als Ziel-Laser wird dabei ein HeNe-Laser (27) verwendet, wobei dessen Laserstrahlung über einen Einkoppelmechanismus (21), welcher im einzelnen in Figur 3 dargestellt ist, eingebracht werden kann.

Um die Energie des Laserstrahls des Argon/Fluorid-Excimer-Lasers möglichst verlustfrei an den zu behandelnden Zahn zu führen, und dabei insbesondere die verwendete Optik nicht zu beschädigen, kann dabei keine herkömmliche Optik verwendet werden. Vielmehr weist die Vorrichtung gemäß Figur 1 und Figur 3 bzw. Figur 2 eine Optik auf, welche grundsätzlich aus Quarzglas gefertige Linsen oder Prismen aufweist und dabei dielektrische Spiegel verwendet. Aufgrund der auftretenden Energieverluste ist es dabei nicht möglich, Quarzfaser zu verwenden.

Der zur Applikation der Laserstrahlung an den zu behandelnden Zahn dienende Handgriff (1) ist dabei wie folgt aufgebaut:

Der Handgriff (1) besteht im wesentlichen aus zwei rohrförmigen Griffabschnitten (2, 6), welche aus Aluminium oder Titan gefertigt sind. Der erste Griffabschnitt (2) besitzt dabei etwa eine Länge von 10 cm, wobei am vorderen Anfang dieser Griffabschnitt (2) rechtwinklig abgeknickt ist und in einem etwa 2 cm hohen Rohrteil zur Bildung eines Optikkopfes (3) ausläuft. In diesem Ende ist eine kurz brennweitige (f = ca. 10 - 12 mm) bikonvexe Quarzlinse (Suprasil I) eingebaut. In der 90°-Abwinklung, welche als Abschrägung der Außenwand des dortigen Rohrteiles ausgebildet ist, ist ein dielektrischer Spiegel (7) angebracht, der für die 193 nm UV-Strahlung optimiert ist und eine Reflektivität von 99,5 % für diese Strahlung bei einem Einfallswinkel von 45° besitzt. Die in Längsrichtung durch die Mitte des ersten Griffabschnittes (2) verlaufende Laserstrahlung gelangt dabei unter einem Einfallswinkel von 45° auf den entsprechend zur Längsrichtung des Griffabschnittes (2) positionierten dielektrischen Spiegel (7) und wird dann unter einem entsprechenden Ausfallswinkel von 45° auf die Mitte der Quarzlinse (13) des Optikkopfes (3) reflektiert.

Das Ende des Rohrteiles zur Aufnahme des Optikkopfes (3) läuft dabei konisch zu und besitzt dort einen Außendurchmesser von 8 mm. Die bikonvexe Quarzlinse (13) ist dabei in einer Ringnut (16) des Optikkopfes (3) gelagert, wobei über einen Feststellring (17) die Quarzlinse (13) gegen einen oberhalb der Kreisnut (16) umlaufenden Radialansatz (18) festlegbar ist. Die Quarzlinse (13) ist insofern gegen eine Wechseloptik, etwa für sichtbares Licht, oder gegen eine andere Quarzlinse mit größerer Brennweite austauschbar.

Der zweite rohrförmige Griffabschnitt (6) des Handgriffes (1) ist am rechten äußeren Ende (5) des ersten rohrförmigen Griffabschnittes (2) angelegt und mit diesem über einen Schwenkmechanismus (10) in Form eines Storchenschnabelmechanismusses verbunden. Die beiden Griffabschnitte (2, 6) sind dabei in ihrer Grundstellung um 90° abgewinkelt, wobei in dieser Abwinklung ein zweiter dielektrischer Spiegel (8) angelegt ist, welcher das vom Eingang (26) durch das Innere des zweiten rohrförmigen Griffabschnittes eintretende Laserlicht unter einem Einfallswinkel von 45° un einem Ausfallswinkel von 45° auf den Optikkopf (3) angelegten dielektrischen Spiegel (7) ablenkt.

Die beiden Griffabschnitte (2, 6) sind dabei über den Schwenkmechanismus (10) und den diese beiden Griffabschnitte an diesen Abschnitten verbindenden Federbalg (11) kippbar miteinander verbunden, wobei eine Verschwenkung der zueinander um 90° abgewinkelten Griffabschnitte um einen Öffnungswinkel $\alpha$ = 35° möglich ist. Da die beiden Schenkel des Storchenschnabelmechanismusses sich immer gleichzeitig öffnen und der dielektrische Spiegel (8) in den Drehpunkten der Griffabschnitte (2, 6) bzw. des Storchenschnabelmechanismusses angeordnet ist, ist sichergestellt, daß bei einer Öffnung des Schwenkmechanismusses (10) der Reflektionspunkt der Laserstrahlung auf der Oberfläche des dielektrischen Spiegels (8) stets an der gleichen Stelle bleibt. Insofern ist es nach einer Neigung des Handgriffes bzw. des ersten oder zweiten Griffabschnittes (2, 6) nicht notwendig, den Laserstrahl neu zu justieren.

Der dielektrische Spiegel (8) ist dabei an dem Storchenschnabelmechanismus derart angebracht, daß die Justierung des eine Reflektivität von 99,5 % bei einem Einfallswinkel von 45 ° für eine Strahlung von 193 nm besitzenden Spiegels möglich ist.

Der zweite Griffabschnitt (2) weist dabei ebenfalls eine Länge von ca. 10 cm auf, wobei der Handgriff zur Zahnbehandlung im allgemeinen an diesem äußeren zweiten Griffabschnitt ergriffen wird. Der erste Griffabschnitt wird dagegen so gehalten, daß dieser an die Stelle des zu behandelnden Zahnes im Mundbereich führt.

Die beiden Griffabschnitte (2, 6) weisen jeweils zwei etwa 5 cm lange Rohrstück-Teile (2a, b, 6a, b) auf, welche jeweils ein um 360° drehbares Drehgelenk (4, 9) bilden. Diese Drehgelenke sind dabei, wie auch die schwenkbare Verbindung der beiden Griffabschnitte im Bereich des Storchenschnabelmechanismusses (10), druckdicht ausgebildet, wobei innerhalb des Handgriffes ein Vakuum erzeugt werden kann und sich somit die Absorption der ultravioletten Laserstrahlung (193 nm) des Argon/Fluorid-Excimer-Lasers erheblich verringert.

Die An/Ausschaltung des Argon/Fluorid-Excimer-Lasers (20) bzw. des Ziel-Lasers (27) erfolgt dabei über einen entsprechenden Ein/Ausschalter (19), welcher an dem äußeren zweiten Griffabschnitt (6) angebracht ist.

Mit Hilfe des Handgriffes (1) ist es möglich, mit den gleichen Freiheitsgraden eine Zahnbehandlung durchzuführen, wie dies bei herkömmlichen mechanischen Zahnbohrern möglich ist. Der Handgriff (1) der Applikationsvorrichtung kann dabei in seiner Abmessung, insbesondere in der Länge der ersten und zweiten Griffabschnitte noch erheblich verkürzt werden, so daß auch bzgl. Abmessung und Gewicht dieser Handgriff nicht größer oder schwerer ist als übliche Handgriffe elektrisch angetriebener mechanischer Bohrer für die Zahnbehandlung.

Die in Figur 2 wiedergegebene zweite Ausführungsform der Applikationsvorrichtung zum Einspiegeln und zum Applizieren der Laserstrahlung des Argon/Fluorid-Excimer-Lasers (193 nm) stellt insofern eine vereinfachte Ausführungsform der in Figur 1 abgebildeten Applikationsvorrichtung bzw. Handgriffes (1) dar, als der erste und zweite Griffabschnitt (2', 6') nicht gänzlich als rohrförmige, nach außen geschlossene Rohre ausgebildet sind. Vielmehr ist der zweite Griffabschnitt (6') über seine gesamte Länge offen ausgebildet und wird dabei lediglich durch den zum Eingang (26') dieser Applikationsvorrichtung sich erstreckenden Bügel (28) des Storchenschnabelmechanismusses (10') begrenzt. Auf der Seite des um 90° zu dem zweiten Griffabschnitt (6') abgewinkelten ersten Griffabschnittes (2') ist dabei dieser Griffabschnitt über einen Anfangsbereich ebenfalls offen geführt, und dabei lediglich seitlich durch einen Bügel (29) des Storchenschnabelmechanismusses begrenzt. Dieser Bügel (29) ist dabei an seinem abgewinkelten Ende auf einen rohrförmigen Abschnitt (30) des ersten Griffabschnittes (2') geführt. Innerhalb dieses Rohrabschnittes ist dabei im Gegensatz zur Ausführungsform der Applikationsvorrichtung gemäß Figur 1 ein Kondensorsystem aus zwei bikonvexen Suprasillinsen (14, 15) angelegt. Durch dieses Kondensorsystem ist es möglich, die Impuls-Energiedichte der auf die Quarzlinse (13') im Optikkopf (3') über einen dielektrischen Spiegel (7') reflektierten UV-Strahlung (193 nm) bedeutend zu erhöhen. Insofern ist es dann möglich, einen Argon/Fluorid-Excimer-Laser mit einer Ausgangs-Impuls-Energiedichte zu benutzen, welche geringer als 200 mJ/cm$^2$ ist.

Ein wesentlicher Vorteil dieses Handgriffes besteht ferner darin, daß dieser über einen Großteil der Griffabschnitte (2, 6) offen ist, und insofern die Handhabung, insbesondere in kleineren, engeren Bereichen, sich einfacher gestaltet.

Die Ausbildung des in Figur 1 angedeuteten Einkoppelmechanismusses (21) für die Strahlung des Argon/Fluorid-Excimer-Lasers (20) und/oder des HeNe-Lasers (27) ist schematisch in Figur 3 dargestellt.

Die aus dem Ausgangsfenster des Excimer-Lasers (20) austretende Strahlung wird dabei vorzugsweise in einer unter Vakuum gesetzten Glasröhre auf einen dielektrischen Spiegel (22) unter einem Einfallswinkel von 45° eingestrahlt und gelangt von diesem Spiegel (22) unter Reflektion unter einem Winkel von 90° auf den Eingang (26) des Handgriffes.

Die Abbildung des im sichtbaren Bereich rotes Licht emittierenden HeNe-Lasers (27) erfolgt dabei über eine entsprechende Optikanordnung, wobei dieses Licht zunächst unter einem Einfallswinkel von 45° auf einen dielektrischen Spiegel (22) fällt und dort aufgrund der Transparenz des dielektrischen Spiegels für sichtbares Licht durchgelassen wird, so daß es schließlich in den Eingang (26) des Handgriffes gelangt. Da der Einkoppelmechanismus (21) dagegen für die 193 nm-Strahlung des Argon/Fluorid-Excimer-Lasers (20) als dielektrischer Spiegel ausgebildet ist, wird das von diesem seitlich auf die Unterseite der dielektrischen Spiegelfläche einfallende Licht dort reflektiert und auf diese Weise dort in den Eingang (26) des Handgriffes reflektiert.

Es ist damit die Laserstrahlung des HeNe-Lasers (27) und die Strahlung des Argon/Fluorid-Excimer-Lasers (20) mittels der Quarzoptik des Handgriffes (1) bzw. (1') gleichzeitig in die Fokusfläche (25) abbildbar.

Über den Optikkopf (3, 3') ist es dabei möglich, neben der Fokusfläche der UV-Strahlung des Argon/Fluorid-Excimer-Lasers, bzw. alternativ an dieser Stelle eine sichtbare Fokusfläche des als Ziel-Laser verwendeten HeNe-Lasers (27) zu erzeugen.

Die in Figur 4 dargestellte Ausführungsform der Applikationsvorrichtung entspricht im wesentlichen der Applikationsvorrichtung (1') gemäß Figur 2. Sie weist dabei ebenfalls einen Storchenschnabelmechanismus (10') sowie ein Kondensorsystem aus Quarzlinsen (Suprasillinsen) auf. Durch das Kondensorsystem wird insofern ebenfalls ein auf einem äußerst engen Querschnitt kollimierter Laserstrahl erzeugt, welcher ebenfalls durch einen Optikkopf auf eine Fokusfläche abgebildet wird.

Im Gegensatz zum Optikkopf (3') wird bei der Ausführungsform gemäß Figur 4 ein Optikkopf (31) verwendet, welcher am Anfang des ersten rohrförmigen Griffabschnittes (2') als gradliniger, in Längsrichtung dieses ersten Griffabschnittes sich fortsetzender Ansatz ausgebildet ist. Im Gegensatz dazu ist der Optikkopf (3) gemäß Figur 1 bzw. (3') gemäß Figur 2 vom rohrförmigen Griffabschnitt nach unten um einen Winkel von 90° abgewinkelt.

Der Optikkopf (31) wird dabei an dem ersten rohrförmigen Griffabschnitt (2') über einen lösbaren Anschluß (32) festgelegt und ist insofern gegen den Optikkopf (3') auswechselbar.

Der Optikkopf (31) ist dabei speziell zum Bohren von Löchern in Zahnhartsubstanzen mittels der 193 nm-Strahlung des Argon/Fluorid-Excimer-Lasers (20) ausgelegt. Er geht dabei hinter dem Anschluß (32) in einen nadelförmigen Abschnitt (33) über, welcher eine Länge von 12 mm und eine Außendurchmesser von 2 mm aufweist. Innerhalb dieses nadelförmigen Abschnittes ist ein Leiterkanal (34) angelegt, in welchen der auf einen Außendurchmesser von ca. 1,59 mm kollimierte Laserstrahl geführt wird. Die gesamte Länge der Applikationsvorrichtung gemessen von der Spitze des nadelförmigen Abschnittes (34) bis zu dem äußeren Bügel (28) des Storchenschnabelmechanismusses (10') beträgt dabei 100 mm. Der Außendurchmesser des zylinderförmigen Griffabschnittes (2') beträgt dabei 12 mm.

Die Applikationsvorrichtung gemäß Figur 4 eignet sich dabei bei Verwendung in einer Vorrichtung mit einem Argon/Fluorid-Excimer-Laser gemäß vorliegender Erfindung im übrigen nicht nur für die Zahnbehandlung, sondern auch unmittelbar für eine Operation am Auge und anderen extern erreichbaren Körperpartien. Als Beispiele seien genannt: In der Augenheilkunde: Hornhautchirurgie, Astigmatismuskorrektur, radiale Keratomie; in der Dermatologie: Resorption bzw. Entfernung kleiner und mittlerer Tumore, wie z. B. Melanome oder Warzen. Der nadelförmige Abschnitt bildet insofern eine etwa 12 mm lange und 2 mm breite Hohlnadel, welche es erlaubt, unmittelbar am Gewebe im externen Körperbereich Operationen vorzunehmen.

Die angedeuteten Operationen lassen sich dabei ebenfalls bereits bei einer angelieferten Ausgangsimpuls-Energiedichte des Argon/Fluorid-Excimer-Lasers (20) von 50 mJ/cm$^2$ ausführen, wobei die Impulsrate ebenfalls kleiner als 100 Hz zu wählen ist, um Verbrennungen der Substanzen zu vermeiden. Ein angelieferter Laserstrahl mit einer Ausgangsimpuls-Energiedichte von 200 mJ/cm$^2$ mit einer Abmessung von 12 x 10 mm wird dabei durch das Kondensorsystem der Applikationsvorrichtung auf eine punktförmige Fokusfläche kollimiert, welche in ihrem Durchmesser durch Anlage einer Quarzlinse am Ende des nadelförmigen Abschnittes (33) bzw. des Leiterkanals (34) noch auf einen bedeutend geringeren Durchmesser fokussierbar ist. In vorteilhafter Weise werden im übrigen Vorsätze unmittelbar außen vor dem Optikkopf zum Einschub von Blenden angelegt.

Da zur Durchführung dieser Operationen ebenfalls keine hohen Energieeinbußen des Argon/Fluorid-Excimer-Lasers in Kauf genommen werden dürfen, muß dabei die Laserstrahlung an dem Operationsort ebenfalls mittels Prismen und Linsen aus Quarz oder dielektrischen Spiegeln gelangen. Es ist dabei aber möglich, den Leiterkanal (34) am Ende statt durch eine Quarzlinse unmittelbar durch ein kurzes Stück (1 mm) einer Quarzfaser abzuschließen. Insofern muß dann die auf eine punktförmige Fokusfläche zu kollimierende Laserstrahlung unmittelbar bereits bei Austritt aus dem Kondensorsystem auf einen derart engen Strahlendurchmesser kollimiert sein.

Zur Applikation von Laserstrahlung für Operationen im Augeninnern ist dabei die Applikationsvorrichtung wie folgt aufgebaut:
Der Handgriff (1'') besteht aus einem ca. 12 mm durchmessenden rohrförmigen ersten Griffabschnitt (2') aus Aluminium oder Titan. Am vorderen Anfang dieses Griffabschnittes ist dabei die Injektionsnadel (33) von ca. 12 mm Länge angelegt, wobei die Dicke dieser Nadel ca. 2 mm beträgt. Am Ende dieses Griffabschnittes (2') befindet sich dabei der Storchenschnabelmechanismus (10'), über welchen der in seiner Grundstellung um 90° abgewinkelte zweite Griffabschnitt (6') verschwenkbar ist. Die Laserstrahlung des Argon/Fluorid-Excimer-Lasers (20) wird dabei in Längsrichtung dieses zweiten Griffabschnittes (6') eingespiegelt. Dazu ist dieser Griffabschnitt an dem Delivery-System des Argon/Fluorid-Excimer-Lasers angeflanscht.

Mit Hilfe des Storchenschnabelmechanismusses (10') und eines im Drehpunkt angebrachten dielektrischen Spiegels (8') (optimiert für 193 nm, 45° Einfallswinkel, 99 % Reflektivität) ist es möglich, den Handgriff um ca. 30° aus der abgewinkelten Grundstellung von 90° zu neigen, ohne den Laserstrahl zu dejustieren.

Da der angelieferte Laserstrahl eine Abmessung von 12 x 10 mm hat, ist es notwendig, ihn vor dem Einkoppeln in die Hohlnadel (33) auf einen Durchmesser von konstant ca. 2 mm zu kollimieren. Dies geschieht mittels eines im Rohr befindlichen Kondensors, der aus zwei plankonvexen Linsen aus Quarzglas (Suprasil) besteht. Dabei beträgt die Brennweite $f_1$ der Linse (14) 60 mm und die Brennweite $f_2$ der Linse (15) 10 mm. Der so kollimierte Laserstrahl wird dann durch die Hohlnadel (33) bzw. deren Leiterkanal (34) geleitet und an das Operationsgebiet gebracht. Die Injektionsnadel weist dabei an ihrem Ende eine Quarzlinse oder eine Saphirlinse (Brennweite f ca. 1 - 2 mm, Durchmesser ca. 1, 8 - 2 mm) auf oder ist durch ein kurzes Stück (1 mm) einer Quarzfaser abgeschlossen.

## Übersicht der Bezugsziffern

| | |
|---|---|
| Handgriff bzw. Applikationsvorrichtung | (1) |
| erster rohrförmiger Griffabschnitt | (2) |
| davon am Anfang abgewinkelter Optikkopf | (3) |
| erstes Drehgelenk | (4) |
| äußeres rechtes Ende des ersten Griffabschnittes | (5) |
| zweiter rohrförmiger Griffabschnitt des Handgriffes | (6) |
| dielektrischer Spiegel innerhalb der Abwinklung des ersten Griffabschnittes | (7) |
| dielektrischer Spiegel in dem schwenkbaren, abgewinkelten Verbindungsabschnitt zwischen erstem und zweitem Griffabschnitt | (8) |
| zweites Drehgelenk | (9) |
| Schwenkmechanismus in Form eines Storchenschnabelmechanismusses | (10) |
| Federbalg | (11) |
| linker Anfang des zweiten rohrförmigen Griffabschnittes | (12) |
| drehgelenkig verbundene "Rohrstück-Teile" des ersten Griffabschnittes | (2a, b) |
| drehgelenkig verbundene "Rohrstückteile" des zweiten Griffabschnittes | (6a, b) |
| bikonvexe Quarzlinse im Optikkopf (3) | (13) |
| Suprasillinsen für Kondensorsystem vor dem Optikkopf (3) | (14, 15) |
| Kreisnut | (16) |
| Feststellring | (17) |
| umlaufender Radialansatz | (18) |
| Ein/Ausschalter am Handgriff für den Laserstrahl | (19) |
| Argon/Fluorid-Excimer-Laser bzw. Ausgangsfenster davon | (20) |
| Einkoppelmechanismus für den Argon/Fluorid-Excimer-Laser und einen HeNe-Laser | (21) |

9

```
dielektrischer Spiegel                                    (22)
durch den Optikkopf erzeugte Fokusfläche der
Laserstrahlung                                            (25)
Eingang des Handgriffes für Laserstrahlung    (26)
He-Ne-Laser                                              (27)


2. Ausführungsform:
Handgriff                                                (1'); (1'')
erster oder zweiter Griffabschnitt          (2', 6')
Eingang des Handgriffes                        (26')
Storchenschnabelmechanismus                   (20')
Bügel des Storchenschnabelmechanismusses    (28, 29)
rohrförmiger Abschnitt des ersten Griff-
abschnittes                                              (30)
Optikkopf                                                (3')
dielektrischer Spiegel                      (7', 8')
Quarzlinse                                               (13')
Federbalg                                                (11')
Optikkopf                                                (31)
Anschluß für einen Optikkopf                (32)
nadelförmiger Abschnitt des Optikkopfes (31)   (33)
Leiterkanal des nadelförmigen Abschnittes   (34)
evakuierte Röhre                                     (35)
```

**Patentansprüche**

1. Vorrichtung zu ablativen Photodekomposition von biologischen Substanzen, welche einen gepulsten Argon/Fluorid-Excimer-Laser aufweist, der mit einer Impulsdauer von 8 - 35 nsec UV-Strahlung einer Wellenlänge von 193 nm bei einer Impulsrate kleiner als 100 Hz emittiert und eine Ausgangsimpuls-Energiedichte von mindestens 50 mJ/cm$^2$ besitzt, sowie mit einer Applikationsvorrichtung zur Leitung der UV-Strahlung des Excimer-Lasers zum Applikationsbereich, wobei das Laserlicht mittels der Applikationsvorrichtung über einen Applikationsarm, in welchem ein Spiegel- und Linsensystem für das UV-Licht angeordnet ist, auf den Applikationsbereich unter Fokussierung auf eine Fläche von kleiner/gleich 2 mm$^2$ geleitet ist,
**dadurch gekennzeichnet, daß**
zur Ermöglichung einer ablativen Photodekomposition von anorganischen Zahnhartsubstanzen im Fokus eine Impuls-Energiedichte von mindestens 2500 mJ/mm$^2$ erzeugt ist.

2. Vorrichtung nach Anspruch 1,
**dadurch gekennzeichnet, daß**
diese des weiteren einen sichtbares Licht emittierenden Ziel-Laser (27) aufweist, welcher in den Strahlengang der Applikationsvorrichtung (1) für das Excimer-Laserlicht in dieses oder neben dieses

10

bzw. dessen Fokus einspiegelbar ist.

3. Vorrichtung nach Anspruch 2,
   **dadurch gekennzeichnet,** daß
   als Ziel-Laser ein HeNe-Laser (27) vorgesehen ist, wobei die Strahlung dieses Ziel-Lasers und des Argon/Fluorid-Excimer-Lasers (20) über einen Einkoppelmechanismus (21) gleichzeitig in den Eingang (26) der Applikationsvorrichtung (1) jeweils einkoppelbar ist.

4. Vorrichtung nach den Ansprüchen 1 - 3,
   **dadurch gekennzeichnet**, daß
   die Impuls-Energiedichte im Fokus größer als 5000 mJ/mm$^2$ ist und die Impulsrate 20 - 50 Hz bei einer Impulsdauer von 15 - 20 nsec beträgt.

5. Vorrichtung nach Anspruch 4,
   **dadurch gekennzeichnet,** daß
   die Impulsrate 25 Impulse/sec beträgt.

6. Vorrichtung nach einem der Ansprüche 1 - 5,
   **dadurch gekennzeichnet,** daß
   der Handgriff des Applikationsarmes mindestens aus einem rohrförmigen Griffabschnitt (2) besteht, in dessen Mitte in Längsrichtung die Laserstrahlung geleitet und in einem am Anfang nach unten abgewinkelten Beleuchtungskopf (2) mit einer bikonvexen oder plankonvexen Quarzlinse (13) über einen dielektrischen Spiegel (7) auf die Fläche ≤ 2 mm$^2$ fokussierbar ist, wobei der rohrförmige Griffabschnitt (2) in einem Abschnitt des nicht nach unten abgewinkelten Teils ein Drehgelenk (4) aufweist und mit einem zweiten nach oben abgewinkelten Griffabschnitt (6) derart über einen Schwenkmechanismus (10) verschwenkbar verbunden ist, und in dieser Abwinklung ein dielektrischer Spiegel (8) derart angeordnet und im Drehpunkt des Gelenkes des Schwenkmechanismusses (10) gelagert ist, daß eine Verstellung der zueinander abgewinkelten Griffabschnitte (2, 6) um einen Schwenkwinkel ($\alpha$) möglich ist, ohne daß der Laserstrahl dadurch dejustiert wird und somit der Reflektionspunkt der Laserstrahlung auf dem dielektrischen Spiegel (8) stets an der selben Stelle ist.

7. Vorrichtung nach Anspruch 6,
   **dadurch gekennzeichnet,** daß
   die beiden Griffabschnitte (2, 6) jeweils rohrförmig ausgebildet sind und in ihrem Inneren Vorrichtungen zur Führung des optischen Strahlenganges in Form dielektrischer Spiegel (7, 8), Linsen, Prismen und Spiegel aus Quarzglas bzw. Calciumfluorid oder solcher Materialien aufweisen, welche durch das hochenergetische UV-Licht des Excimer-Lasers nicht zerstört werden und eine hohe Reflektivität bei dem vorgegebenen Einfallswinkel bzw. hohe Transmissionen aufweisen.

8. Vorrichtung nach Anspruch 6 oder 7,
   **dadurch gekennzeichnet,** daß
   beide Griffabschnitte (2, 6) rohrförmig sind und jeweils ein Drehgelenk (4, 9) aufweisen.

9. Vorrichtung nach einem der Ansprüche 6, 7 oder 8,
   **dadurch gekennzeichnet,** daß
   die Drehgelenke (4, 9) jeweils eine relative Verstellung der entsprechenden Teile der Griffabschnitte (2, 9) um 360° ermöglichen.

10. Vorrichtung nach einem der Ansprüche 6 - 9,
    **dadurch gekennzeichnet,** daß
    als Schwenkmechanismus (10) am Ende (5) des ersten rohrförmigen Griffabschnittes (2) ein Storchenschnabelmechanismus (10) vorgesehen ist.

11. Vorrichtung nach einem der Ansprüche 1 - 10,
    **dadurch gekennzeichnet,** daß
    gegeneinander bewegbare Griffabschnitte über einen Federbalg (11) aus Metall miteinander verbunden sind.

**12.** Vorrichtung nach Anspruch 11,
**dadurch gekennzeichnet,** daß
der Federbalg (11) das Ende (5) des ersten Griffabschnittes mit dem Anfang (12) des zweiten Griffabschnittes (6) verbindet und über die gesamte Umfangrichtung vakuumdicht ausgebildet ist.

**13.** Vorrichtung nach einem der Ansprüche 6 - 12,
**dadurch gekennzeichnet,** daß
der erste rohrförmige Griffabschnitt (2) nach unten rechtwinklig oder unter einem stumpfen Winkel abgewinkelt ist, wobei in der Abwinklung der dielektrische Spiegel (7) angeordnet ist, von welchem nach Reflektion des UV-Lichtes des Ecximer-Lasers dieses auf eine kurz-brennweitige Quarzlinse (13) geworfen wird, welche eine Spotgröße des Lasers von $\leq 2$ mm$^2$ erzeugt.

**14.** Vorrichtung nach Anspruch 13,
**dadurch gekennzeichnet,** daß
die im ersten und/oder zweiten rohrförmigen Griffabschnitt (2, 6) ein Kondensorsystem aus Suprasillinsen (14, 15) angeordnet ist.

**15.** Vorrichtung nach einem der vorstehenden Ansprüche 1 - 14,
**dadurch gekennzeichnet,** daß
als bikonvexe oder plankonvexe Linse (13) im Optikkopf (3) des ersten armförmigen Griffabschnittes (2) eine Suprasil- oder Saphir-Linse mit einer Brennweite von 10 - 15 mm verwendet ist.

**16.** Vorrichtung nach einem der Ansprüche 1 - 15,
**dadurch gekennzeichnet,** daß
die dielektrischen Spiegel (7, 8) auf eine Reflektivität von 99,5 % bei dem vorgegebenen Einfallswinkel (z. B. 45° gemäß Figur 1, 2) für die vorgegebene Wellenlänge von 193 nm optimiert sind.

**17.** Vorrichtung nach einem der Ansprüche 1 - 19,
**dadurch gekennzeichnet,** daß
der Optikkopf (3) der Applikationsvorrichtung (1) eine bikonvexe oder plankonvexe Linse (13) aufweist, welche zur Fokussierung des Argon/Fluorid-Excimer-Laserlichts und zur Transmission dieses Lichtes optimiert ist und/oder eine mit dieser Linse (13) austauschbare Optik für einen Ziel-Laser oder einen zur Aussteuerung des Lasers dienenden weiteren optischen Laser aufweist.

**18.** Vorrichtung nach einem der Ansprüche 1 - 17,
**dadurch gekennzeichnet,** daß
der Handgriff (1) bzw. die rohrförmigen Griffabschnitte (2, 6) vakuumdicht miteinander verbunden sind und über eine Vakuumpumpe unter Vakuum setzbar sind, wobei die Übertragung der ultravioletten Laserstrahlung (193 nm) des Argon/Fluorid-Excimer-Lasers (20) zwischen diesem und der Applikationsvorrichtung (1) über eine evakuierte Röhre (35) erfolgt.

**19.** Vorrichtung nach einem der Ansprüche 1 - 18,
**dadurch gekennzeichnet,** daß
zur gleichzeitigen Einblendung der ultravioletten Laserstrahlung des Argon/Fluorid-Excimer-Lasers (20) und der sichtbaren Strahlung eines Ziel-Lasers ein Einkoppelmechanismus (21) verwendet ist, welcher für sichtbares Licht transparent ist und für die 193 nm-Strahlung als dielektrischer Spiegel bedampft ist (99,5 % Reflektivität für 193 nm, bei 45° Einfallswinkel).

**20.** Vorrichtung nach einem der Ansprüche 1 - 19,
**dadurch gekennzeichnet,** daß
das Ende der Applikationsvorrichtung (1), bzw. der erste rohrförmige Griffabschnitt (2), seitlich einen Anschluß (32) für auswechselbare Beleuchtungsköpfe (3, 3', 31) aufweist.

**21.** Vorrichtung nach Anspruch 20,
**dadurch gekennzeichnet,** daß
der Anschluß (32) als Steck-, Schraub- oder Klemmverbindung an der seitlichen Stirnwand des rohrartigen Griffabschnittes (2, 2') angelegt und dort ein nach unten abgewinkelter bzw. abgebogener, oder ein geradlinig in Längsrichtung des ersten Griffabschnittes sich fortsetzender Optikkopf (3, 3', 33) festgesetzt ist.

**22.** Vorrichtung nach Anspruch 21,
**dadurch gekennzeichnet,** daß
ein sich geradlinig in Längsrichtung des ersten rohrförmigen Griffabschnittes (2') erstreckender Optikkopf (3') seitlich von einem Anschlußflansch bzw. Rohrabschnitt als ein sich verjüngender Rohrabschnitt mit einer Quarzoptik (13') oder als ein nadelförmiger Abschnitt (33) mit einem in Längsrichtung zentrisch verlaufenden Leiterkanal (34) für die Laserstrahlung ausgebildet ist.

**23.** Vorrichtung nach Anspruch 22,
**dadurch gekennzeichnet,** daß
der nadelförmige Abschnitt (33) mit einer Spitze als Hohlnadel ausgebildet ist.

**24.** Vorrichtung nach den Ansprüchen 22 oder 23,
**dadurch gekennzeichnet,** daß
der nadelförmige Abschnitt bzw. die Hohlnadel (33) an ihrem Anfang eine Quarzlinse oder Saphirlinse oder zum Abschluß des Leiterkanals (34) ein kurzes Stück Quarzfaser aufweisen.

**25.** Vorrichtung nach den Ansprüchen 22, 23, 24,
**dadurch gekennzeichnet,** daß
in dem rohrförmigen Griffabschnitt (2') außerhalb des Optikkopfes (31) ein den Durchmesser des Strahlenganges der vom Argon/Fluorid-Excimer Laser emittierten Laserstrahlung auf den Innendurchmesser des Leiterkanals (34) kollimierendes Kondensorsystem (14, 15) angelegt ist.

**26.** Vorrichtung nach einem der vorstehenden Ansprüche 22 - 25,
**dadurch gekennzeichnet,** daß
bei einem mit einer Hohlnadel versehenen Optikkopf (31) die Nadeldicke ca. 2 mm, die Nadellänge ca. 12 mm und die Fokusfläche der zur Applikation erzeugten Laserstrahlung im Durchmesser 1,596 mm oder kleiner, bis punktförmig ist.

**27.** Vorrichtung nach einem der Ansprüche 1 - 26,
**dadurch gekennzeichnet,** daß
der Applikationsarm (1) gegenüber der äußeren Atmosphäre abgedichtet und evakuiert ist.

**28.** Vorrichtung nach Anspruch 27,
**dadurch gekennzeichnet,** daß
der Applikationsarm (1) mit Luft oder Stickstoff kühlbar ist.

**29.** Vorrichtung nach einem der Ansprüche 1 - 28,
**dadurch gekennzeichnet,** daß
die Vorrichtung zur ablativen Photodekomposition von Zahnsubstanzen ferner ein Spektralanalyse-System zur Differenzierung der gesunden Zahnsubstanzen, wie Dentin, Schmelz, von kariös veränderten derartigen Zahnsubstanzen aufweist, wobei Steuervorrichtungen vorgesehen sind, welche eine Aussteuerung bzw. An- und Ausschaltung des Excimer-Lasers über die Änderung der vom Excimer-Laser oder einer zusätzlichen Lichtquelle (Hg-Lampe) induzierten Fluoreszenz bewirken.

## Claims

**1.** Device for ablative photodecomposition of biological substances, which device exhibits a pulsed argon/fluoride excimer laser, which emits UV radiation of a wavelength of 193 nm at a pulse rate lower than 100 Hz with a pulse duration of 8 - 35 nsec, and possesses an output pulse energy density of at least 50 mJ/cm$^2$, and having an application device to conduct the UV radiation of the excimer laser to the application region, the laser light being conducted by means of the application device via an application arm, in which a mirror and lens system for the UV light is disposed, to the application region with focusing onto an area of less than/equal to 2 mm$^2$, characterised in that in order to permit an ablative photodecomposition of inorganic hard dental substances, a pulse energy density of at least 2500 mJ/mm$^2$ is generated at the focus.

2. Device according to Claim 1, characterised in that the device further exhibits a sighting laser (27), which emits visible light and which can be reflected into the beam path of the application device (1) for the excimer laser light, into the latter or beside the latter or its focus.

3. Device according to Claim 2, characterised in that an HeNe laser (27) is provided as sighting laser, the radiation of this sighting laser and of the argon/fluoride excimer laser (20) being capable of being coupled in via a coupling-in mechanism (21) simultaneously into the entrance (26) of the application device (1) in each instance.

4. Device according to Claims 1 - 3, characterised in that the pulse energy density at the focus is greater than 5000 mJ/mm$^2$ and the pulse rate is 20 - 50 Hz with a pulse duration of 15 - 20 nsec.

5. Device according to Claim 4, characterised in that the pulse rate is 25 pulses/sec.

6. Device according to one of Claims 1 - 5, characterised in that the handle of the application arm comprises at least a tubular handle portion (2), in the centre of which the laser radiation is conducted in the longitudinal direction and, in an initially downwardly angled-off illumination head (2), can be focused by a biconvex or planoconvex quartz lens (13) via a dielectric mirror (7) onto the area $\leq$ 2 mm$^2$, the tubular handle portion (2) exhibiting a swivel joint (4) in a portion of the part which is not angled-off downwardly, and being swivellably connected to a second, upwardly angled-off handle portion (6) in such a manner, by means of a swivel mechanism (10), and a dielectric mirror (8) being disposed in such a manner in this angling-off and being mounted at the fulcrum of the joint of the swivel mechanism (10), that a displacement of the mutually angled-off handle portions (2, 6) by a swivel angle ($\alpha$) is possible, without the laser beam being maladjusted thereby and thus the point of reflection of the laser radiation on the dielectric mirror (8) is invariably at the same place.

7. Device according to Claim 6, characterised in that the two handle portions (2, 6) are designed in each instance to be tubular and exhibit in their interior devices for guiding the optical beam path in the form of dielectric mirrors (7, 8), lenses, prisms and mirrors of quartz glass or calcium fluoride or such materials which are not destroyed by the high-energy UV light of the excimer laser and exhibit a high reflectivity at the predetermined angle of incidence or high transmissions respectively.

8. Device according to Claim 6 or 7, characterised in that the two handle portions (2, 6) are tubular and exhibit in each instance a swivel joint (4, 9).

9. Device according to one of Claims 6, 7 or 8, characterised in that the swivel joints (4, 9) permit in each instance a relative displacement of the corresponding parts of the handle portions (2, 9) through 360°.

10. Device according to one of Claims 6 - 9, characterised in that a pantograph mechanism (10) is provided as swivel mechanism (10) at the end (5) of the first tubular handle portion (2).

11. Device according to one of Claims 1 - 10, characterised in that relatively movable handle portions are connected to one another by means of a spring bellows (11) of metal.

12. Device according to Claim 11, characterised in that the spring bellows (11) connect the end (5) of the first handle portion to the start (12) of the second handle portion (6) and is designed to be vacuum-sealed over the entire circumferential direction.

13. Device according to one of Claims 6 - 12, characterised in that the first tubular handle portion (2) is angled-off downwardly at right angles or at an obtuse angle, the dielectric mirror (7) being disposed in the angling-off, from which dielectric mirror, after reflection of the UV light of the excimer laser, said light is thrown onto a short-focus quartz lens (13), which generates a spot size of the laser of $\leq$ 2 mm$^2$.

14. Device according to Claim 13, characterised in that a condenser system of Suprasil lenses (14, 15) is disposed in the first and/or second tubular handle portion (2, 6).

15. Device according to one of the preceding Claims 1 - 14, characterised in that a Suprasil or sapphire lens having a focal length of 10 - 15 mm is used as biconvex or planoconvex lens (13) in the optical

14

head (3) of the first arm-shaped handle portion (2).

16. Device according to one of Claims 1 - 15, characterised in that the dielectric mirrors (7, 8) are optimised to a reflectivity of 99.5% at the predetermined angle of incidence (e.g. 45° according to Figure 1, 2) for the predetermined wavelength of 193 nm.

17. Device according to one of Claims 1 - 19, characterised in that the optical head (3) of the application device (1) exhibits a biconvex or planoconvex lens (13), which is optimised for the focusing of the argon/fluoride excimer laser light and for the transmission of this light and/or exhibits an optical system, which is exchangeable with this lens (13), for a sighting laser or a further optical laser employed to control the laser.

18. Device according to one of Claims 1 - 17, characterised in that the handle (1) or the tubular handle portions (2, 6) are connected to one another in a vacuum-sealed manner and can be placed under vacuum by means of a vacuum pump, the transmission of the ultraviolet laser radiation (193 nm) of the argon/fluoride excimer laser (20) between said laser and the application device (1) taking place via an evacuated tube (35).

19. Device according to one of Claims 1 - 18, characterised in that, for the simultaneous merging of the ultraviolet laser radiation of the argon/fluoride excimer laser (20) and the visible radiation of a sighting laser, a coupling-in mechanism (21) is used, which is transparent to visible light and has a vapour-deposited coating as dielectric mirror for the 193 nm radiation (99.5% reflectivity for 193 nm, at an angle of incidence of 45°).

20. Device according to one of Claims 1 - 19, characterised in that the end of the application device (1), or of the first tubular handle portion (2), exhibits laterally a connection (32) for exchangeable illumination heads (3, 3', 31).

21. Device according to Claim 20, characterised in that the connection (32) is constructed as a plug connection, screw connection or compression connection on the lateral end wall of the tubular handle portion (2, 2') and a downwardly angled-off or offset optical head (3, 3' 33) or an optical head continuing in a straight line in the longitudinal direction of the first handle portion is fixed there.

22. Device according to Claim 21, characterised in that an optical head (3') extending in a straight line in the longitudinal direction of the first tubular handle portion (2') is designed laterally from a connecting flange or tube portion as a tapering tube portion with a quartz optical system (13') or as a needle-shaped portion (33) with a guide channel (34), extending centrally in the longitudinal direction, for the laser radiation.

23. Device according to Claim 22, characterised in that the needle-shaped portion (33) is designed with a tip as a hollow needle.

24. Device according to Claims 22 or 23, characterised in that the needle-shaped portion or the hollow needle (33) exhibits at its start a quartz lens or sapphire lens or, to terminate the guide channel (34), a short piece of quartz fibre.

25. Device according to Claims 22, 23 or 24, characterised in that a condenser system (14, 15) collimating the diameter of the beam path of the laser radiation emitted by the argon/fluoride excimer laser to the internal diameter of the guide channel (34) is constructed in the tubular handle portion (2') outside the optical head (31).

26. Device according to one of the preceding Claims 22 - 25, characterised in that in the case of an optical head (31) provided with a hollow needle, the needle thickness is approximately 2 mm, the needle length is approximately 12 mm and the focus area of the laser radiation generated for application is 1.596 mm or smaller in diameter, down to a point configuration.

27. Device according to one of Claims 1 - 26, characterised in that the application arm (1) is sealed off in relation to the external atmosphere and evacuated.

15

**28.** Device according to Claim 27, characterised in that the application arm (1) can be cooled by air or nitrogen.

**29.** Device according to one of Claims 1 - 28, characterised in that the device for the ablative photo-decomposition of dental substances further exhibits a spectral analysis system for the differentiation of the healthy dental substances, such as dentene, enamel, from such dental substances exhibiting changes due to caries, control devices being provided, which effect a control or energisation and deenergisation of the excimer laser by means of the alteration of the fluorescence induced by the excimer laser or an additional light source (Hg lamp).

**Revendications**

**1.** Dispositif pour la photodécomposition ablative de substances biologiques, qui présente un laser Excimer à l'argon/fluorure pulsé, qui émet, avec une durée d'impulsions de 8-35 nanosecondes, un rayonnement UV d'une longueur d'onde de 193 nm avec un taux d'impulsions inférieur à 100 Hz et qui possède une intensité d'énergie d'impulsion de sortie d'au moins 50 mJ/cm$^2$, et qui comprend un dispositif d'application pour guider le rayonnement UV du laser Excimer vers le domaine d'application, dans lequel la lumière laser est guidée sur le domaine d'application par focalisation sur une surface inférieure ou égale à 2 mm$^2$, au moyen du dispositif d'application à l'intervention d'un bras d'application dans lequel est installé un système de miroirs et de lentilles pour la lumière UV, caractérisé en ce que, pour rendre possible une photodécomposition ablative de substances dentaires dures inorganiques, on produit dans le foyer une intensité d'énergie d'impulsion d'au moins 2.500 mJ/mm$^2$.

**2.** Dispositif selon la revendication 1, caractérisé en ce que ce dernier présente, en outre, un laser cible (27) émettant de la lumière visible, qui peut s'introduire par réflexion dans la marche des rayons du dispositif d'application (1) pour la lumière du laser Excimer, dans cette dernière ou à côté de cette dernière ou du foyer de cette dernière.

**3.** Dispositif selon la revendication 2, caractérisé en ce que, comme laser cible, on prévoit un laser HeNe (27), le rayonnement de ce laser cible et celui du laser Excimer (20) à l'argon/fluorure pouvant être respectivement alimentés simultanément dans l'entrée (26) du dispositif d'application (1) à l'intervention d'un mécanisme d'alimentation (21).

**4.** Dispositif selon les revendications 1-3, caractérisé en ce que la densité d'énergie d'impulsion dans le foyer est supérieure à 5.000 mJ/mm$^2$ et le taux d'impulsions s'élève à 20-50 Hz avec une durée d'impulsions de 15-20 nsec.

**5.** Dispositif selon la revendication 4, caractérisé en ce que le taux d'impulsions s'élève à 25 impulsions/sec.

**6.** Dispositif selon l'une quelconque des revendications 1-5, caractérisé en ce que la poignée du bras d'application est constituée d'au moins une section de préhension tubulaire (2), au milieu de laquelle le rayonnement laser est guidé en direction longitudinale et peut être focalisé sur la surface ≤ 2 mm$^2$ dans une tête d'éclairage (3) formant au début un angle tourné vers le bas munie d'une lentille à quartz (13) biconvexe ou plan-convexe, à l'intervention d'un miroir diélectrique (7), la section de préhension tubulaire (2) présentant une liaison articulée (4) dans une section de la partie qui ne forme pas un angle dirigé vers le bas et étant reliée en pivotement à l'intervention d'un mécanisme (10) destiné à cet effet à une seconde section de préhension (6) formant un angle tourné vers le haut, et un miroir diélectrique (8) étant disposé dans cette section angulaire et monté dans le pivot de l'articulation du mécanisme de pivotement (10), tout ceci de telle sorte qu'un déplacement des sections de préhension (2, 6) angulairement opposées soit possible en formant un angle de pivotement ($\alpha$) sans que le rayonnement laser s'en trouve déréglé et qu'ainsi, le point de réflexion du rayonnement laser sur le miroir diélectrique (8) se trouve toujours au même endroit.

**7.** Dispositif selon la revendication 6, caractérisé en ce que les deux sections de préhension (2, 6) sont réalisées respectivement en une forme tubulaire et présentent, en leur sein, des dispositifs pour le guidage de la marche des rayons optiques sous la forme d'un miroir diélectrique (7, 8), de lentilles, de prismes et de miroirs en verre quartzeux ou en fluorure de calcium, ou encore présentent des matières telles qu'elles ne sont pas détruites par la lumière UV à haute énergie du laser Excimer et présentent une réflectivité élevée dans l'angle d'incidence prédéfini ou encore des transmissions élevées.

8. Dispositif selon la revendication 6 ou 7, caractérisé en ce que les deux sections de préhension (2, 6) sont de forme tubulaire et présentent, respectivement, une liaison articulée (4, 9).

9. Dispositif selon l'une quelconque des revendications 6, 7 ou 8, caractérisé en ce que les liaisons articulées (4, 9) rendent respectivement possible un déplacement relatif des parties correspondantes des sections de préhension (2, 9) de 360°.

10. Dispositif selon l'une quelconque des revendications 6 à 9, caractérisé en ce que, comme mécanisme de pivotement (10), on prévoit un mécanisme de pantographe (10) à l'extrémité (5) de la première section de préhension tubulaire (2).

11. Dispositif selon l'une quelconque des revendications 1 à 10, caractérisé en ce que des sections de préhension à mobilité antagoniste sont reliées l'une à l'autre à l'intervention d'un soufflet (11) en métal.

12. Dispositif selon la revendication 11, caractérisé en ce que le soufflet (11) relie la fin (5) de la première section de préhension au début (12) de la seconde section de préhension (6) et est réalisé pour être étanche au vide sur toute sa périphérie.

13. Dispositif selon l'une quelconque des revendications 6 à 12, caractérisé en ce que la première section de préhension tubulaire (2) forme un angle droit dirigé vers le bas ou bien forme un angle obtus, le miroir diélectrique (7) étant disposé dans la section angulaire, à partir duquel, après réflexion de la lumière UV du laser Excimer, celle-ci est projetée sur une lentille à quartz (13) à courte distance focale qui procure une grandeur ponctuelle du laser de $\leq 2 \text{ mm}^2$.

14. Dispositif selon la revendication 13, caractérisé en ce qu'un système de condenseur constitué par des lentilles Suprasil (14, 15) est disposé dans la première et/ou la seconde section de préhension tubulaire (2, 6).

15. Dispositif selon l'une quelconque des revendications 1 à 14, caractérisé en ce que, comme lentille biconvexe ou plan-convexe (13) dans la tête d'optique (3) de la première section de préhension (2) en forme de bras, on utilise une lentille Suprasil ou à saphir avec une distance focale de 10-15 mm.

16. Dispositif selon l'une quelconque des revendications 1 à 15, caractérisé en ce que les miroirs diélectriques (7, 8) sont optimisés à une réflectivité de 99,5% dans l'angle d'incidence prédéfini (par exemple de 45° selon les figures 1, 2) pour la longueur d'onde prédéfinie de 193 nm.

17. Dispositif selon l'une quelconque des revendications 1 à 19, caractérisé en ce que la tête d'optique (3) du dispositif d'application (1) présente une lentille biconvexe ou plan-convexe (13) qui est optimisée pour la focalisation de la lumière du laser Excimer à l'argon/fluorure et pour la transmission de cette lumière, et/ou présente une optique qui peut remplacer cette lentille (13) pour un laser cible ou encore un autre laser optique servant au réglage du laser.

18. Dispositif selon l'une quelconque des revendications 1 à 17, caractérisé en ce que la poignée (1) ou les sections de préhension tubulaires (2, 6) sont reliées l'une à l'autre de manière étanche au vide et peuvent être alimentées en vide à l'intervention d'une pompe à vide, la transmission du rayonnement laser ultraviolet (193 nm) du laser Excimer (20) à l'argon/fluorure ayant lieu entre ce dernier et le dispositif d'application (1) à l'intervention d'un tube (35) dans lequel on a fait le vide.

19. Dispositif selon l'une quelconque des revendications 1 à 18, caractérisé en ce que, pour l'introduction simultanée en mélange du rayonnement laser ultraviolet du laser Excimer (20) à l'argon/fluorure et du rayonnement visible d'un laser cible, on utilise un mécanisme d'alimentation (21) qui est transparent pour la lumière visible et qui est métallisé au vide en forme de miroir diélectrique pour le rayonnement de 193 nm (réflectivité de 99,5% pour 193 nm avec un angle d'incidence de 45°).

**20.** Dispositif selon l'une quelconque des revendications 1 à 19, caractérisé en ce que la fin du dispositif d'application (1) ou de la première section de préhension tubulaire (2) présente latéralement un raccord (32) pour des têtes d'éclairage remplaçables (3, 3', 31).

**21.** Dispositif selon la revendication 20, caractérisé en ce que le raccord (32) est disposé contre la paroi frontale latérale de la section de préhension tubulaire (2, 2') en forme de liaison par enfichage, par vissage ou par serrage et, à cet endroit, on fixe une tête d'optique (3, 3', 33) formant un angle ou une courbure dirigée vers le bas ou encore faisant saillie de manière rectiligne en direction longitudinale par rapport à la première section de préhension.

**22.** Dispositif selon la revendication 21, caractérisé en ce qu'une tête d'optique (3') s'étendant de manière rectiligne en direction longitudinale de la première section de préhension tubulaire (2') latéralement par rapport à une bride de raccordement ou à une section tubulaire, est réalisée en une section tubulaire se rétrécissant munie d'une optique à quartz (13') ou bien en une section aciculaire (33) munie d'un canal conducteur (34) s'étendant en position centrale en direction longitudinale, destiné au rayonnement laser.

**23.** Dispositif selon la revendication 22, caractérisé en ce que la section aciculaire (33) est réalisée à l'aide d'une pointe en forme d'aiguille creuse.

**24.** Dispositif selon la revendication 22 ou 23, caractérisé en ce que la section aciculaire ou l'aiguille creuse (33) présente, à leur début, une lentille à quartz ou une lentille à saphir ou encore un petit morceau de fibre à quartz pour la fermeture du canal conducteur (34).

**25.** Dispositif selon les revendications 22, 23, 24, caractérisé en ce que, dans la section de préhension tubulaire (2') à l'extérieur de la tête d'optique (31), on applique un système de condenseur (14, 15) collimatant le diamètre de la marche des rayons du rayonnement laser émis par le laser Excimer à l'argon/fluorure pour obtenir le diamètre interne du canal conducteur (34).

**26.** Dispositif selon l'une quelconque des revendications précédentes 22 à 25, caractérisé en ce que, dans le cas d'une tête d'optique (31) munie d'une aiguille creuse, l'épaisseur de l'aiguille est d'environ 2 mm, la longueur de l'aiguille est d'environ 12 mm et la surface de foyer du rayonnement laser produit pour l'application a un diamètre de 1,596 mm ou moins jusqu'à un diamètre ponctuel.

**27.** Dispositif selon l'une quelconque des revendications 1 à 26, caractérisé en' ce qu'on fait le vide dans le bras d'application (1) et en ce qu'on lui procure une étanchéité vis-à-vis de l'atmosphère externe.

**28.** Dispositif selon la revendication 27, caractérisé en ce que le bras d'application (1) peut être refroidi avec de l'air ou de l'azote.

**29.** Dispositif selon l'une quelconque des revendications 1 à 28, caractérisé en ce que le dispositif pour la photodécomposition ablative de substances dentaires présente, en outre, un système d'analyse spectrale pour la différenciation des substances dentaires saines telles que la dentine, l'émail, par rapport à des substances dentaires de ce type ayant subi une modification par carie, des dispositifs de commande étant prévus, qui mettent en oeuvre un réglage ou une mise en circuit et hors-circuit du laser Excimer à l'intervention de la modification de la fluorescence induite par le laser Excimer ou par une source lumineuse supplémentaire (lampe Hg).

18

FIG.1

FIG.2

Kondensorsystem
Suprasillinsen

LASERSTRAHL

Storchenschnabelmechanismus

Dielektrischer
Spiegel (99% R)

1'
6'
26'
28
10
8'
11'
2'
29
30
15
14
7'
3'
13'

FIG. 3

Kondensorsystem
Suprasillinsen

LASERSTRAHL

FIG. 4